# EUROPÄISCHE PATENTSCHRIFT

(11) **EP 3 806 920 B1**
(45) Veröffentlichungstag und Bekanntmachung des Hinweises auf die Patenterteilung: **06.04.2022**
(21) Anmeldenummer: 19733413.9
(22) Anmeldetag: 13.06.2019
(51) Int. Cl.: A61L 31/04, A61B 5/0215, A61B 5/00, A61B 8/04, A61L 31/08, A61L 31/14

(54) **MEDIZINISCHES IMPLANTAT, ANORDNUNG ZUM IMPLANTIEREN DES MEDIZINISCHEN IMPLANTATS SOWIE ANORDNUNG ZUM ERFASSEN EINES INTRAKORPORALEN BEWEGUNGSMUSTERS MIT DEM MEDIZINISCHEN IMPLANTAT**
MEDICAL IMPLANT, ASSEMBLY FOR IMPLANTING THE MEDICAL IMPLANT, AND ASSEMBLY FOR DETECTING AN INTRACORPOREAL MOVEMENT PATTERN USING THE MEDICAL IMPLANT
IMPLANT MÉDICAL, DISPOSITIF POUR IMPLANTER L'IMPLANT MÉDICAL AINSI QUE DISPOSITIF POUR DÉTECTER UN MODÈLE DE MOUVEMENT INTRACORPOREL À L'AIDE DE L'IMPLANT MÉDICAL

(30) Priorität: 13.06.2018 DE 102018209449
(43) Veröffentlichungstag der Anmeldung: 21.04.2021
(73) Patentinhaber: Neuroloop GmbH, 79110 Freiburg (DE)
(72) Erfinder: PLACHTA, Dennis, 79279 Vörstetten (DE)
(74) Vertreter: Rösler, Uwe
(86) Internationale Anmeldenummer: PCT/EP2019/065441
(87) Internationale Veröffentlichungsnummer: WO 2019/238803

(56) Entgegenhaltungen:
- WO-A1-2006/122750
- WO-A1-2018/024868
- DE-T2- 69 907 475

## Beschreibung

### Technisches Gebiet

Die Erfindung bezieht sich auf eine aus wenigstens einem biokompatiblen Material gefertigte Struktur, einer Anordnung zum Implantieren der Struktur sowie einer Anordnung zum Erfassen eines intrakorporalen Bewegungsmusters mit der Struktur. Insbesondere gilt es mit Hilfe der Struktur wenigstens einen physiologischen Parameter, vorzugsweise den menschlichen oder tierischen Blutdruck zu erfassen.

### Stand der Technik

Die messtechnische Erfassung physiologischer Parameter ist in der Medizintechnik üblich und weit verbreitet. Ein Beispiel für eine derartige Erfassung eines physiologischen Parameters ist die kontinuierliche Messung des arteriellen Blutdrucks. Ein derartiges Gerät und zugehöriges Erfassungsverfahren, die ohne die bekannte aufpumpbare Armmanschette mit dem nach dem Riva-Rocci-Prinzip funktionierenden Drucksensor auskommt, sind bspw. in der Druckschrift US 5,241,964, beschrieben. Dabei werden zwei nichtinvasive Ultraschall-Doppler-Sensoren extrakorporal über einer größeren Arterie angebracht. Die damit gemessenen Blutflußsignale werden anschließend herangezogen, um ein vereinfachtes mathematisch-empirisches Modell der Arterie parametrisch zu charakterisieren. Die dabei ermittelte sogenannte Resonanzfrequenz des Modells ist mit dem Blutdruck korreliert. Eine herkömmliche Manschettenmessung kalibriert in unregelmäßigen Abständen das System auf den Blutdruck.

In der Druckschrift DE 198 29 544 C1 wird ebenfalls eine Vorrichtung zur nichtinvasiven Blutdruckmessung beschrieben. Mittels Ultraschall- oder Laser-Dopplertechnik wird z.B. eine mit dem Blutfluss oder der Blutflussgeschwindigkeit verknüpfte Größe gemessen. Die der Messwerterfassung nachgeschaltete Signalverarbeitung umfasst eine Filterung zur Beseitigung von Artefakten und sonstigen Störungen.

Problematisch für eine möglichst exakte Blutdruckerfassung mittels optischer oder Ultraschall-basierter Pulswellensensoren ist zum einen die Sensibilität bezüglich einer korrekten räumlichen extrakorporalen Anbringung und Ausrichtung der jeweiligen Sensoren relativ zu den blutführenden Gefäßregionen. Bereits geringfügige Dejustierungen in der Sensorausrichtung relativ zu einem blutführenden Gefäß können eine zuverlässige Blutdruckerfassung verunmöglichen. Zum anderen sind die zur Blutdruckerfassung relevanten, detektierbaren Signalpegel nur sehr gering und bedürfen daher einer aufwendigen Verstärkung, Filterung und Signalauswertung. Dies erfordert system- und kostenaufwendige Anstrengungen.

Die Druckschrift WO 2018/024868 A1 offenbart eine Vorrichtung zur Blutdruckmessung mit Hilfe von Ultraschallwellen, die einen intrakorporalen Träger aufweist, an den wenigstens eine stationäre Ultraschallreflektorfläche sowie wenigstens eine bewegliche Ultraschallreflektorfläche angeordnet sind. Letztere vermag sich in Abhängigkeit des schwankenden Blutdruckes zu bewegen. Mit Hilfe einer extrakorporalen Ultraschallwandlereinheit kann die beweglich angeordnete Ultraschallreflektorfläche relativ zur stationär angeordneten Ultraschallreflektorfläche detektiert werden.

Die Druckschrift DE 699 07 475 T2 offenbart eine implantierbare Vorrichtung, die echoerzeugendes Material enthält, vorzugsweise in Form von 100 nm bis 500 nm großer Teilchen, das für eine verbesserte Sichtbarkeit auf einem Ultraschall-Scan dienen soll.

Die Druckschrift WO 2006/122750 A1 offenbart einen implantierbaren Blutdrucksensor, der aus einem gewebeähnlichen Material in Art eines Rings gefertigt ist, der ein blutführendes Gefäß umfasst und durch die Blutdruckschwankungen dilatiert bzw. verformt wird. In den ringförmigen Träger sind Dehnungsmessstreifen eingebracht, die die blutdruckbedingten Deformationen erfassen und deren Messsignale extrakorporal übertragen werden.

### Darstellung der Erfindung

Der Erfindung liegt die Aufgabe zugrunde, die vorstehend genannten Nachteile bei der Erfassung medizinisch relevanter physiologischer Parameter, so insbesondere bei der Erfassung des Blutdruckes, eines Menschen oder eines Tieres durch transkutanes Einschallen von Ultraschallwellen sowie Detektieren intrakorporal reflektierter Ultraschallwellenanteile weitgehend abzustellen. So gilt es zum einen die Robustheit der vorzunehmenden Messung gegen Dejustierungen der zum Aussenden und Empfang von Ultraschallwellen erforderlichen Komponenten signifikant zu verbessern und zum anderen gilt es Maßnahmen zu ergreifen, den messtechnischen Aufwand sowie auch den mit der Signalauswertung verbundenen Aufwand zu reduzieren.

Eine lösungsgemäße Vorrichtung ist Gegenstand des Anspruches 1, der sich auf eine aus wenigstens einem biokompatiblen Material gefertigte Struktur bezieht, die mit Hilfe einer Anordnung gemäß Anspruch 12 intrakorporal applizierbar ist. Gegenstand des Anspruches 14 ist eine Anordnung zum Erfassen eines intrakorporalen Bewegungsmusters mit Hilfe der Struktur, wodurch es möglich ist medizinisch relevante physiologische Parameter zu erfassen. Den Erfindungsgedanken in vorteilhafter Weise weiterbildende Merkmale sind Gegenstand der Unteransprüche sowie der weiteren Beschreibung insbesondere unter Bezugnahme auf die Ausführungsbeispiele zu entnehmen.

Die der Erfindung zugrundeliegende Idee betrifft eine aus biokompatiblen Material bestehende Struktur, die mit Hilfe einer Kanülenanordnung transkutan intrakorporal, vorzugsweise in unmittelbare Nähe zu einem blutführenden Gefäß implantierbar ist und sich innerhalb des Körpers selbständig und gegebenenfalls durch Applikation äußerer Kraft- oder Energiefelder oder durch fluidmechanische Unterstützung derart zu entfalten vermag, so dass die Struktur eine so genannte effektive Wirkoberfläche ausbildet, an der Ultraschallwellen reflektieren. Die aus biokompatiblem Material gefertigte Struktur verfügt darüber hinaus über eine Eigenelastizität, die mit der Eigenelastizität organischer Gefäßwände vergleichbar ist, so insbesondere jener der blutführenden Gefäßwand, relativ zu der die implantierte Struktur angeordnet wird. Durch die Materialwahl der biokompatiblen Struktur sowie deren geometrische Ausbildung und intrakorporale Anordnung gegenüber eines blutführenden Gefäßes unterliegt die biokompatible Struktur und insbesondere deren effektive Wirkoberfläche räumlichen Deformationen, die durch natürliche Pulswellen bedingt sind, die wiederum durch den pulsatilen Blutdruck innerhalb des blutführenden Gefäßes und der sich pulsatil weitenden und zusammenziehenden Gefäßwand in die unmittelbar an die Gefäßwand extravasal angrenzenden Körperbereiche übertragen werden. Durch die makroskopisch gewählte Größe der effektiven Wirkoberfläche vermag diese einen durchaus beträchtlichen Anteil von mit Hilfe eines extrakorporal platzierten Sonographiegerätes subkutan eingeschallten Ultraschallwellen zu reflektieren. Hierdurch werden die Anforderungen an eine exakte räumliche Ausrichtung des Sonographiegerätes relativ zum blutführenden Gefäß im Gegensatz zu den bekannten Verfahren, die eine direkte Erfassung des blutführenden Gefäßes erfordern, erheblich gesenkt. Zudem rufen die an der effektiven Wirkoberfläche reflektierten Ultraschallwellen im Sonographiegerät Ultraschallsignale mit einem weitaus höheren Signalpegel hervor, wodurch das S/N-Verhältnis verbessert und damit der Aufwand in der nachfolgenden Signalprozessierung und -auswertung reduziert werden kann.

Die lösungsgemäße aus wenigstens einem biokompatiblen Material gefertigte Struktur zeichnet sich somit durch die Kombination der nachfolgenden Merkmale aus: Die aus biokompatiblen Material gefertigte Struktur ist aus einem ersten räumlich kompakten Zustand in einen zweiten räumlich ausladenden, flexibel verformbaren Zustand überführbar, in dem die ausladende Struktur eine effektive Wirkoberfläche besitzt, die flexibel verformbar ist und Ultraschallwellen zumindest teilweise zu reflektieren vermag. Das biokompatible Material der Struktur verfügt über ein Elastizitätsmodul, das dem der Gefäßwand eines blutführenden Gefäßes entsprich und in der Größenordnung zwischen 10⁵Nm⁻² und 10⁷Nm⁻² liegt. Zudem verfügt die Struktur zumindest in einem Bereich über eine akustische Impedanz, die größer als 1,63 x 10⁶ kg/m²s ist. Im zweiten Zustand, in dem die aus biokompatiblen Material gefertigte Struktur einen räumlich ausladenden Formzustand annimmt besitzt die Struktur eine effektive Wirkoberfläche, die flexible verformbar ist und Ultraschallwellen zumindest teilweise zu reflektieren vermag.

Die Struktur ist in Art einer Folie, eines Geflechtes, einer Schwamm- oder Knäuelstruktur ausgebildet und nimmt aufgrund ihrer materialelastischen Eigenschaften im ersten Zustand vorzugsweise in eine ball- oder kapselförmige Raumform an, die ein Einführen sowie auch Durchführen durch eine medizinische Hohlkanüle ermöglicht, die eine Kanülengröße zwischen 10G und 30G, vorzugsweise 17G und 25G besitzt. Dies bedeutet, dass die im ersten Zustand kompaktierte Struktur eine Durchmesserdimension aufweist, die dem Innendurchmesser der Hohlkanüle entspricht. In geeigneter Weise nimmt die Struktur im ersten Zustand eine gestreckt ellipsoide oder zylinderförmige Raumform an, deren Zylinderdurchmesser dem Innendurchmesser der jeweils gewählten Hohlkanüle entspricht und in dieser Form vermittels eines Mandrins oder eines druckbeaufschlagbaren Mittels durch die Hohlkanüle distalseits geschoben oder befördert werden kann. Anstelle der Verwendung eines Mandrins ist es denkbar die Hohlkanüle proximalseitig mit einer druckbeaufschlagten Flüssigkeit, bspw. Kochsalzlösung, zu beaufschlagen, um die kompaktierte Struktur innerhalb der Hohlkanüle durch die distale Öffnung zu fördern.

Vorzugsweise entfaltet sich die Struktur aus ihrem kompaktierten bzw. komprimierten ersten Zustand in den zweiten Zustand aufgrund materialinhärenter elastischer Rückstellkräfte selbständig. Der Vorgang des Entfaltens bzw. des sich Vergrößerns in den expandierten bzw. entfalteten Zustand kann durch zusätzliche äußere Einwirkungen unterstützt werden. So erfolgt der Vorgang des sich Vergrößerns vom ersten in den zweiten Zustand innerhalb des intrakorporalen, feuchten Milieus, das den "Entfaltungsvorgang" zu unterstützen vermag. In einem bevorzugten Ausführungsbeispiel verfügt das biokompatible Material über hygroskopische Eigenschaft und vermag durch Wasseraufnahme die Strukturentfaltung bzw. -ausdehung wirkungsvoll zu unterstützen. Alternativ oder in Kombination mit der vorstehend beschriebenen Maßnahme wird durch Applikation eines externen Kraft- oder Energiefeldes, das mit Hilfe eines extrakorporal angeordneten Kraftfelderzeugers generierbar ist, eine den Umformungsvorgang vom ersten in den zweiten Zustand unterstützende Wirkung erzielt. In einem bevorzugten Ausführungsbeispiel weist hierzu die aus biokompatiblen Material gefertigte Struktur wenigstens ein Mittel und/oder eine materielle Eigenschaft auf, wodurch die Struktur mittel- oder unmittelbar in Wechselwirkung mit dem extrakorporal applizierten Kraftfeld tritt, so dass ein die Raumform und/oder die räumliche Lage der Struktur veränderliches Kraftmoment erzeugbar ist, das den Transformationsvorgang vom ersten in den zweiten Zustand zu unterstützen vermag und/oder eine Positionierung der intrakorporal applizierten und in den zweiten Zustand übergeführten Struktur ermöglicht.

Je nach Ausprägung des Kraftfeldes, beispielsweise in Form eines Magnetfeldes, elektrischen Feldes, schallakustischen Feldes oder eines kalorischen Feldes, d.h. eines Temperaturfeldes, ist das an der Struktur anzubringende oder in dieser integrierte Mittel geeignet auszuwählen. Im Falle eines Magnetfeldes eignen sich magnetische oder magnetisierbare Materialanteile, die in oder an die Struktur ein- oder angebracht sind. Im Falle eines elektrischen Feldes dienen elektrisch leitende Materialabschnitte innerhalb der Struktur, die in Wechselwirkung mit einem elektrodynamischen oder elektrostatischen Feld treten. Im Falle eines schallakustischen Kraftfeldes dienen an der Struktur lokal angebrachte Mittel, die Schallwellen bevorzugt absorbieren oder reflektieren, um ein durch Schallimpulsübertragung auf die Struktur wirkendes Kraftmoment funktional nutzen zu können. Im Falle eines extrakorporal applizierten kalorischen Kraftfeldes, eignen sich bspw. bimetallische oder bimetallartig wirkende Materialien oder Materialkombinationen, die in der Struktur integriert oder an dieser angebracht zu lokalen Strukturverformungen führen können.

Auch eignen sich Wandlermaterialien oder so genannte Smart Materials, bspw. Materialien mit Formgedächtnis, zur Integration bzw. Applikation in oder auf der Struktur, um mit Hilfe eines extern applizierten Kraftfeldes Verformungskräfte zu generieren.

Geeignete Materialien für die Realisierung eines lösungsgemäßen Implantats stellen beispielsweise auch Metamaterialien oder hybride Materialkombinationen aus Metamaterial und biologischen Gewebematerial und/oder bioverträglichen Polymeren dar. Auch sind folienartige Substrate aus oder zumindest mit oberflächig belegten Nanostrukturen bspw. in Form von Nanotubes oder Nanograss geeignet, Derartige zumeist aus Kohlenstoff oder Titanlegierungen (bspw. Ti6Al4V, TiO2)) gefertigte Nanostrukturen verfügen über speziell konditionierbare physikalische Eigenschaften, die sich in Gegenwart von Energiefeldern in geeigneter Weise beeinflussen lassen.

Für alle zusätzlichen an der Struktur additiv oder integrativ hinzugefügten Materialien gilt das für medizinische Implantate geltende Auswahlkriterium der Bioverträglichkeit bzw. Biokompatibilität.

Typischerweise liegt die Struktur initial im zweiten Zustand vor, d.h. in einem räumlich ausladenden, nicht komprimierten Zustand bzw. in einer nicht kompaktierten Form. Ausgehend von diesem Zustand wird die Struktur vorzugsweise durch Falten, Komprimieren und/oder Wickeln in den ersten Zustand überführt und zum Zwecke der Implantation in eine Hohlkanüle platziert.

Nach entsprechender Separation der Struktur aus der Hohlkanüle entfaltet sich bzw. expandiert die Struktur und bildet die effektive Wirkoberfläche aus, an der Ultraschallwellen reflektieren. Die effektive Wirkoberfläche weist eine Flächengröße von wenigstens 0,2 mm² und maximal 500 mm² auf. Die vorzugsweise flächig einstückig zusammenhängende, effektive Wirkoberfläche wird im implantierten Zustand relativ zu einem blutführenden Gefäß derart orientiert, so dass die von dem blutführenden Gefäß ausgehenden Pulswellen die effektive Wirkoberfläche möglichst orthogonal durchschallen, d.h. die Pulswellenausbreitungsrichtung schließt mit der effektiven Wirkoberfläche vorzugsweise einen Winkel α von 90° ± 30° ein. Auf diese Weise wird die effektive Wirkoberfläche der implantierten Struktur in Abhängigkeit der Pulswellen dynamisch verformt.

Treffen diagnostische Ultraschallwellen auf die effektive Wirkoberfläche, die mit Hilfe eines an sich bekannten Sonographiegerätes über die Haut, d.h. subkutan eingeschallt werden, so wird wenigstens ein Teil der auf die effektive Wirkoberfläche auftreffenden Ultraschallwellen reflektiert und mit Hilfe des Sonographiegerätes empfangen. Die vom Sonographiegerät empfangenen Ultraschallwellen enthalten Informationen über den sich jeweils zeitlich ändernden Verformungszustand der effektiven Wirkoberfläche der implantierten Struktur, der jeweils mit dem innerhalb des blutführenden Gefäßes vorherrschenden Blutdruck korreliert ist. Im Rahmen einer Messwert-Kalibrierung, die bspw. mit Hilfe einer bekannten aufpumpbaren Armmanschette mit einem nach dem Riva-Rocci-Prinzip funktionierenden Drucksensor durchgeführt wird, können die mit Hilfe des Sonographiegerätes gewonnenen Ultraschallsignale quantitativen Blutdruckwerten zugeordnet werden.

In einer weiteren bevorzugten Ausführungsform ist die aus biokompatiblen Material bestehende Struktur im Unterschied zu einem vorstehend erläuterten flächigen, folienartigen Substrat, in Art eines Geflechtes, einer Schwamm- oder Knäuelstruktur ausgebildet, die im räumlich expandierten zweiten Zustand eine effektive Wirkoberfläche besitzt, die sich in Projektion längs einer Raumrichtung, die idealerweise mit der Ausbreitungsrichtung der Ultraschallwellen zusammenfällt, auf die expandierte Struktur im zweiten Zustand darstellt. Bspw. im Falle einer sphärischen Knäuelstruktur stellt die effektive Wirkoberfläche eine Kreisfläche dar mit einem Durchmesser, der dem Durchmesser der räumlichen Knäuelstruktur entspricht.

Nicht notwendigerweise jedoch in vorteilhafter Form ist die effektive Wirkoberfläche strukturiert und vorzugsweise wellig oder zickzackartige geformt, wobei die vorgegebene Strukturierung trotz der Pulswellen bedingten Deformation im Wesentlichen erhalten bleibt und dadurch in die an der effektiven Wirkoberfläche reflektierten Ultraschallwellen eine typische Signatur einprägt, wodurch eine weitgehend fehlerfreie Signaldetektion ermöglicht wird und Fehlersignalanteile, die von möglichen intrakorporalen Störreflexen herrühren und keine derartige charakteristische Signatur aufweisen, erkannt und bei der Signalauswertung nihiliert werden können.

Um zu gewährleisten, dass die implantierte Struktur im zweiten Zustand nach entsprechender Entfaltung und räumlicher Positionierung möglichst unverändert am Ort verbleibt und nicht unkontrolliert zu vagabundieren beginnt, sieht ein bevorzugtes Ausführungsbeispiel wenigstens ein Verankerungselement an der Struktur vor, das sich mechanisch am unmittelbar umliegenden Gewebe zu verankern vermag. Beispielsweise ist das Verankerungselement in Art eines widerhakenförmigen Abschnittes mittel- oder unmittelbar an der Struktur ausgebildet.

Eine weitere bevorzugte Ausführungsform sieht an der Struktur zusätzliche elektromagnetische Wellen, vorzugsweise in Form von Radarwellen, reflektierende Materialbereiche vor. Hierdurch wird die Funktion der Struktur als reiner Ultraschallwellenreflektor um eine weitere Funktion ergänzt, der die Wechselwirkung mit elektromagnetischen Wellen zugrunde liegt.

In Kombination oder alternativ zu den vorstehenden vorteilhaften Ausführungsformen sieht die Struktur wenigstens einen Strukturbereich mit einer RFID-, Interdigitalelektroden- und/oder elektrischen Spulen-Struktur vor, über die jeweils elektrische Energie sowie auch signalbasierte Informationen kontaktlos zwischen der implantierten Struktur und einer extrakorporalen Sende- und Empfangseinheit übertragen werden können.

In einer weiteren bevorzugten Ausführungsform dient die implantierbare Struktur auch als Träger für wenigstens einen funktionellen Stoff, der an die aus biokompatiblen Material gefertigte Struktur appliziert und in dieser integriert sein kann. Der funktionale Stoff ist vorzugsweise ein pharmazeutischer Wirkstoff, der im implantierten Zustand der Struktur, vorzugsweise unter Vorgabe eines bestimmten Dosierungsmusters lokal abgegeben werden kann. Die Wirkstoffabgabe kann zeitlich definiert vorgegeben und/oder vermittels extrakorporal applizierbarer Kraftfelder individuell beeinflusst werden.

Alternativ oder im Kombination mit einem pharmazeutischen Wirkstoff dient in einer weiteren Ausführungsform ein bioverträglicher Adhäsionskleber als funktionaler Stoff zur Immobilisierung der Struktur und dauerhaften Verortung an einer bestimmten intrakorporalen Stelle mit einer definiert vorgegebenen räumlichen Lage. Der biokompatible Adhäsionskleber ist derart an der Struktur appliziert, so dass der Kleber im Zustand der räumlich expandierten Struktur oberflächig in Kontakt mit benachbarten Gewerbeoberflächen tritt, an denen sich eine dauerhafte Fügeverbindung ausbildet.

Wie bereits vorstehend erwähnt dient hygroskopisches Material, das als funktionaler Stoff in die aus bioverträglichem Material bestehende Struktur eingebracht ist, durch Aufnahme von Gewebsflüssigkeit als ein die Entfaltung bzw. Expansion unterstützendes Mittel.

Zum Zwecke des Implantierens der Struktur dient eine Hohlkanüle die derart angepasst und geeignet zur subkutanen Implantation der Struktur ausgebildet ist, so dass die im ersten Zustand befindliche Struktur, innerhalb der Hohlkanüle ein- bzw. untergebracht werden kann, um sie nachfolgend distalseitig aus der Hohlkanüle intrakorporal durch die Haut hindurch zu applizieren. Vorzugsweise dient hierzu ein als Mandrin ausgebildetes Schubmittel, mit dem die innerhalb der Hohlkanüle platzierte Struktur durch einen Operateur präzise distalseitig aus der Hohlkanüle geschoben und platziert werden kann. Alternativ zur Verwendung eines Mandrins ist proximalseitig an die Hohlkanüle ein druckbeaufschlagbares Flüssigkeitsreservoir anschließbar, über das eine bioverträgliche Flüssigkeit, beispielsweise eine Salzlösung, die innerhalb der Hohlkanüle platzierte Struktur druckbeaufschlagt distalwärts fördert, um diese letztendlich intrakorporal zu applizieren. Die zusätzlich über die Hohlkanüle intrakorporal dosiert abgebbare Flüssigkeitsmenge vermag die Entfaltung bzw. Expansion der komprimierten Struktur intrakorporal zu unterstützen und wird im Weiteren vom umliegenden Gewebe resorbiert.

Verfügt die Struktur in einer vorstehend erläuterten, bevorzugten Ausführungsform über ein Mittel, das in Wechselwirkung mit einem externen bzw. extrakorporal applizierten Kraftfeld zu treten vermag, so ist ein extrakorporal anzuordnender Generator erforderlich, der ein Kraftfeldes der nachfolgenden Art zu erzeugen vermag: magnetisches Feld, elektrisches Feld, kalorisches Feld und/oder schallakustisches Feld. Vorzugsweise vermag der Generator das Kraftfeld in Bezug auf Feldstärke und/oder räumliche Feldverteilung variabel erzeugen.

Die lösungsgemäße Struktur stellt eine Teilkomponente einer Anordnung zum Erfassen eines intrakorporalen Bewegungsmusters dar, die zudem ein an sich bekanntes Sonographiegerät vorsieht, das extrakorporal derart zu positionieren ist, dass die vermittels des Sonographiegerätes erzeugten Ultraschallwellen auf die effektive Wirkoberfläche der intrakorporal applizierten Struktur treffen und an dieser zumindest teilweise reflektieren. Durch Detektion der an der effektiven Wirkoberfläche reflektierten Ultraschallwellen sind räumliche Distanzänderungen zwischen der effektiven Wirkoberfläche und dem Sonographiegerät erfassbar, die zur Bestimmung eines intrakorporalen Bewegungsmusters zugrunde gelegt werden. Insbesondere ist es auf diese Weise möglich den Blutdruck nach entsprechender Kalibrierung der mit dem Sonographiegerät gewonnenen Messwerte zu erfassen und dessen zeitliche Veränderung messtechnisch zu verfolgen. Die lösungsgemäße Anordnung ermöglicht somit eine dauerhafte Erfassung und Überwachung des Blutdruckes ohne jegliche unangenehm auf den Patienten wirkende Zusatzkomponenten, wie bspw. Armdruckmanschette.

In vorteilhafter Weise sieht die Anordnung zum Erfassen des intrakorporalen Bewegungsmusters zusätzlich eine Sende- und Empfangseinheit für ein elektromagnetisches Felder vor, die extrakorporal angeordnet ist und deren elektromagnetisches Feld mit einer an der implantierten Struktur angebrachten RFID-, Interdigitalelektroden- und/oder elektrischen Spulen-Struktur in Wechselwirkung tritt.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Figur 1a, b, c: Darstellung einer aus biokompatiblem Material gefertigten Struktur für ein medizinisches Implantat,
- Fig. 2: Anordnung zum Implantieren des medizinischen Implantats,
- Fig. 3a, b: Ausführungsvariante zum Entfalten des medizinischen Implantates,
- Fig. 4: medizinisches Implantat mit integrierten Fluidkanälen, sowie
- Fig. 5: Darstellung einer Anordnung zum Erfassen eines intrakorporalen Bewegungsmusters

### Wege zur Ausführung der Erfindung, gewerbliche Verwendbarkeit

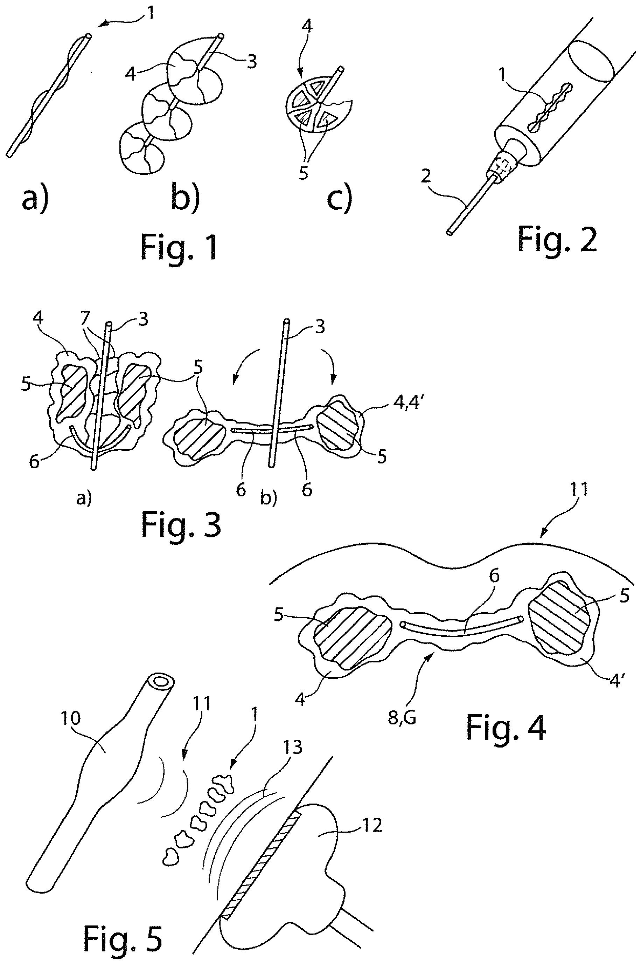

Fig. 1a zeigt die Struktur 1 in einer räumlich komprimierten Raumform, die dafür geeignet ist, die Struktur 1 durch eine Hohlkanüle 2, siehe Figur 2, zum Zwecke einer intrakorporalen Verortung zu platzieren. Fig. 2 stellt eine spritzenartige Vorrichtung zum Einbringen der Struktur 1 mit Hilfe einer Kanüle 2 in einen menschlichen oder tierischen Körper (nicht dargestellt) dar. Das Einbringen der Struktur 1 kann mit Hilfe einer Trägerflüssigkeit, beispielsweise Saline, oder in "trockener" Form erfolgen. Die Hohlkanüle 2 sollte im Rahmen des Implantationsvorganges in der Nähe eines größeren, arteriellen Blutgefäßes, möglichst parallel zu diesem Gefäß, zum Zwecke der Injizierung der Struktur 1 orientiert sein.

Die komprimierte Struktur 1 umfasst gemäß Darstellung in Figur 1a einen Stützstab 3, vorzugsweise in Form eines Polysaccharid-Stabes, längs dem eine flächige, folienartige Struktur 4 angebracht, die einstückig oder mehrteilig ausgebildet sein kann. Durch Zurückziehen der Kanüle 2 und Ausstoßen der Struktur 1 entfaltet sich die flächige, folienartige Struktur 4 fächerförmig radial von dem Stützstab 3 gemäß Fig. 1b.

Die flächige, folienartige Struktur 4 besteht vorzugsweise aus einer flexiblen Polymerfolie, an deren Oberfläche vorzugsweise eine Beschichtung 5 aus Ultraschall reflektierendem Material aufgebracht ist. Das Ultraschall reflektierende Material kann überdies zusätzlich auch über elektromagnetische Wellen reflektierende Eigenschaften verfügen. Vorzugsweise eignet sich hierzu Nanostrukturmaterial in Form von Nanotubes oder Nanograss, beispielsweise in Form von Carbonnanotubes oder Titanoxid-Nanograss, siehe auch Fig. 1c.

Für eine verbesserte Auf- bzw. Entfaltung der flächigen Struktur 1 aus dem komprimierten Zustand in den entfalteten Zustand sieht eine in Fig. 3a, b illustrierte Ausführungsform eine mit wenigstens einem Fluidkanal 6, vorzugsweise Ölkanal, durchsetzte flächige, folienartige Struktur 4 des medizinischen Implantates 1 vor. Der vorzugsweise mit Öl befüllte Kanal 6 erzeugt eine mechanische Vorspannung, die in der komprimierten Form des medizinischen Implantats 1 von zusätzlichen Fäden 7 gehalten wird, die die flächigen, folienartigen Strukturen 4 des medizinischen Implantats 1 mit dem Stützstab 3 verbinden, siehe Figur 3a. Sobald sich das medizinische Implantat 1 innerhalb des Körpers befindet, resorbieren die Haltefäden 7, so dass die Fluidkanäle 6 das flächige Substrat des medizinischen Implantats 1 zu entfalten vermögen, siehe Fig. 3b.

Das flächige Substrat 4 des medizinischen Implantats 1 kann einzelne Flächenbereiche 4' aufweisen, die alle oder jeweils paarweise mit einem Fluidkanal 6 verbunden sind. Auf diese Weise ist es möglich, dass sich die einzelnen Flächenbereiche 4' unabhängig voneinander verdreht ausweiten können, was die Ausrichtung der Flächenbereiche in Form von Reflektorflächen und letztlich dem reflektierten Signal zu Gute kommt. Durch eine bevorzugte Befüllung der Kanäle 6 vorzugsweise mit Öl können störende Einflüsse auf das Ultraschallwellen-Reflexionsverhalten des medizinischen Implantates 1 vermieden, zumal Öl für Ultraschall akustisch transparent ist.

Ferner kann davon ausgegangen werden, dass durch das intrakorporale feuchte Milieu aufgrund osmotischer Druckwirkung auf das medizinische Implantat 1 Wasser in und durch das polymerbasierte Flächensubstrat 4 des medizinischen Implantates 1 zu dringen vermag, wodurch in die Kanäle 6 Wasser eindringt und so die Spannkraft der Flächenstruktur des medizinischen Implantates 1 erhöht wird.

Wünschenswert ist es zudem, dass die einzelnen Flächenbereiche 4' des medizinischen Implantates 1 in Abhängigkeit der intrakorporal auftretenden Pulswellen 11 ihre räumliche Ausrichtung und/oder Form variieren. Die räumliche Variation kann dadurch unterstützt und realisiert werden, indem die einzelnen Flächenbereiche 4' des medizinischen Implantats 1 beweglich gegeneinander angeordnet sind. Dies wird durch Engstellen 8 zwischen den zumindest paarweise zusammenhängenden Flächenbereichen 4' gewährleistet, längs den jeweils wenigstens eine Fluidkanal 6 verläuft, siehe Figur 4. Die Engstellen 8 wirken in Form eines "natürlichen Gelenkes" 9, das die mechanische Beweglichkeit der einzelnen Flächenbereiche 4' relativ zueinander vorzugeben vermag. Im Falle des in Figur 4 gezeigten Zustandes ist der Stützstab 3 bereits resorbiert.

Fig. 5 illustriert ein intrakorporales, blutführendes Gefäß 10, von dem Blutdruckwellen 11 ausgehen, die mit dem lösungsgemäßen medizinischen Implantat 1 in Wechselwirkung treten, so dass sich das flächig ausgebreitete, medizinische Implantat 1 durch die Druckwellen 11 räumlich deformiert wird. Mit Hilfe eines extrakorporalen angeordneten Ultraschallkopfes 12 werden subkutan Ultraschallwellen 13 in den Bereich des medizinischen Implantates 1 eingeschallt. Durch die Blutdruckwellen 11 bedingten räumlichen Deformationen am medizinischen Implantat 1 werden die Ultraschallwellen 13 am medizinischen Implantat 1 reflektiert und moduliert Diese Modulation in den reflektierten Ultraschallwellen stellt eine Funktion der Stärke der Deformation bzw. Auslenkung des medizinischen Implantats 1 und damit verbunden eine Stärke der Blutdruckwellen 11 dar, die detektiert und exakt gemessen werden kann.

### Bezugszeichenliste

- 1: Medizinisches Implantat
- 2: Hohlkanüle
- 3: Stützstab
- 4: Folienartiges Substrat
- 4': Flächenbereiche des medizinischen Implantats
- 5: Nanostrukturen
- 6: Hohlkanal
- 7: resorbierbare Haltefäden
- 8: Engstelle
- 9: Gelenk
- 10: blutführendes Gefäß
- 11: Blutdruckwellen
- 12: Ultraschallkoppler
- 13: Ultraschallwellen

## Patentansprüche

1. Aus wenigstens einem biokompatiblen Material gefertigte Struktur, die durch eine Hohlkanüle transkutan, intrakorporal implatierbar ist und die
- in Art einer Folie, eines Geflechtes, einer Schwamm- oder Knäuelstruktur ausgebildet ist,
- sich aus einem ersten räumlich kompakten Zustand selbständig in einen zweiten räumlich ausladenden und flexibel verformbaren Zustand zu entfalten vermag,
- ein Elastizitätsmodul besitzt, das dem einer Gefäßwand eines Blut-führenden Gefäßes in der Größenordnung zwischen 10⁵ N m⁻² und 10⁷ N m⁻² entspricht,
- zumindest einen Bereich mit einer akustischen Impedanz von größer 1,63·10⁶ kg/m²s besitzt,
- im zweiten Zustand eine effektive Wirkoberfläche besitzt, die flexibel verformbar ist und Ultraschallwellen zumindest teilweise reflektiert, und
- im ersten Zustand eine Durchmesserdimension aufweist, die einem Innendurchmesser einer Hohlkanüle mit einer Kanülengröße zwischen 10 G und 30 G entspricht.

2. Struktur nach Anspruch 1,
**dadurch gekennzeichnet, dass** die Struktur wenigstens ein Mittel aufweist, das in Wechselwirkung mit einem extrakorporalen magnetischen, elektrischen, kalorischen und/oder schallakustischen Kraftfeld derart bringbar ist, dass ein die Raumform und/oder die räumliche Lage der Struktur veränderliches Kraftmoment erzeugbar ist.

3. Struktur nach Anspruch 1 oder 2,
**dadurch gekennzeichnet, dass** die Struktur im zweiten Zustand eine effektive Wirkoberfläche von wenigstens 0,2 mm² und maximal 500 mm² besitzt.

4. Struktur nach einem der Ansprüche 1 bis 3,
**dadurch gekennzeichnet, dass** die Struktur ausgehend vom zweiten Zustand durch Falten, Komprimieren und/oder Wickeln in den ersten Zustand überführbar ist.

5. Struktur nach einem der Ansprüche 2 bis 4,
**dadurch gekennzeichnet, dass** das mit dem externen Kraftfeld in Wechselwirkung tretende Mittel aus wenigstens einem Material der nachfolgenden Materialgruppe besteht: magnetisches oder magnetisierbares Material; elektrisch leitendes Material; bi-metallisches Material; thermisch, elektrisch oder magnetisches Wandlermaterial, Materialien mit Formgedächtnis.

6. Struktur nach einem der Ansprüche 1 bis 5,
**dadurch gekennzeichnet, dass** die effektive Wirkoberfläche in Form einer einstückig zusammenhängenden Fläche ausgebildet ist.

7. Struktur nach einem der Ansprüche 1 bis 6,
**dadurch gekennzeichnet, dass** die in Art eines Geflechtes, einer Schwamm- oder Knäuelstruktur ausgebildete Struktur in Projektion längs einer Raumrichtung die für sich längs der Raumrichtung ausbreitenden Ultraschallwellen zumindest teilweise reflektierende effektive Wirkoberfläche bildet.

8. Struktur nach einem der Ansprüche 1 bis 7,
**dadurch gekennzeichnet, dass** wenigstens einer der nachfolgenden Stoffe an den Struktur appliziert oder in dieser integriert ist: pharmazeutischer Wirkstoff, Adhäsionskleber, hygroskopisches Material.

9. Struktur nach einem der Ansprüche 1 bis 8,
**dadurch gekennzeichnet, dass** die Struktur Metamaterialien oder hybride Materialkombinationen aus Metamaterial und biologischem Gewebematerial und/oder bioverträglichen Polymer aufweist.

10. Struktur nach einem der Ansprüche 3 bis 9
**dadurch gekennzeichnet, dass** die Struktur aus Nanostrukturen oder zumindest mit oberflächig belegten Nanostrukturen ausgebildet ist.

11. Struktur nach Anspruch 10,
**dadurch gekennzeichnet, dass** die Nanostrukturen in Form von Nanotubes oder Nanograss ausgebildet ist.

12. Anordnung zum Implantieren der Struktur nach einem der Ansprüche 1 bis 11, umfassend folgende Komponenten:
- Hohlkanüle angepasst und geeignet zur subkutanen Implantation der Struktur, das im ersten Zustand innerhalb der Hohlkanüle untergebracht ist und distalseitig aus der Hohlkanüle intrakorporal subkutan applizierbar ist,
- Mittel zur distalseitigen Abgabe der Struktur aus der Hohlkanüle und intrakorporalen Applikation.

13. Anordnung zum Implantieren nach Anspruch 2 und 12,
**dadurch gekennzeichnet, dass** ein Generator zur Erzeugung des magnetischen, elektrischen, kalorischen und/oder schallakustischen Kraftfeldes vorgesehen ist, das in Wechselwirkung mit dem wenigstens einen Mittel der Struktur tritt.

14. Anordnung zum Erfassen eines intrakorporalen Bewegungsmusters mit der Struktur nach einem der Ansprüche 1 bis 11 sowie einem Sonographiegerät, das extrakorporal derart positioniert ist, dass vermittels des Sonographiegerätes erzeugter Ultraschallwellen, die auf die effektive Wirkoberfläche der intrakorporal applizierten Struktur treffen und an dieser teilweise reflektieren, räumliche Distanzänderungen zwischen der effektiven Wirkoberfläche und dem Sonographiegerät erfassbar sind.

15. Anordnung nach Anspruch 14 sowie Anspruch 5,
**dadurch gekennzeichnet, dass** eine Sende- und Empfangseinheit für elektromagnetische Felder extrakorporal angeordnet ist, deren elektromagnetisches Feld mit der RFID-, Interdigitalelektroden- und/oder elektrische Spulen-Struktur in Wechselwirkung tritt.

## Claims

1. Structure, made of at least one biocompatible material, which is intracorporeally implantable by way of a hollow cannula and which
- is designed in the form of a film, a braid, a sponge or ball structure,
- can unfold by itself from a first spatially compact state into a second spatially expansive and flexibly deformable state,
- has a module of elasticity that corresponds to the vascular wall of a blood-carrying vessel of a magnitude between 10⁵ N m⁻² and 10⁷ N m⁻²,
- has at least one area with an acoustic impedance of more than 1.63·10⁶ kg/m²s,
- in the second state has an effective active surface that is flexibly deformable and at least partially reflects ultrasonic waves, and
- in the first state has a diameter dimension corresponding to an inner diameter of a hollow cannula with a cannula size of between 10 G and 30 G.

2. Structure according to claim 1,
**characterised in that** the structure comprises at least one means which can be brought into interaction with an extracorporeal magnetic, electrical, caloric and and/or acoustic force field in such a way that a force moment changing the spatial shape and/or the spatial position of the structure can be generated.

3. Structure according to claim 1 or 2,
**characterised in that** in the second state the structure comprises an effective active surface of at least 0.2 mm² and maximally 500 mm².

4. Structure according to any one of claims 1 to 3,
**characterised in that** starting from the second state, the structure can be transformed into the first state by folding, compressing and/or wrapping.

5. Structure according to any one of claims 2 to 4,
**characterised in that** the means interacting with the external force field comprises at least one material from the following material group: magnetic or magnetisable material; electrically conductive material; bi-metallic material; thermic, electrical or magnetic transformer material, shape memory materials.

6. Structure according to any one of claims 1 to 5,
**characterised in that** the effective active surface is configured in the form of a one-piece contiguous surface.

7. Structure according to any one of claims 1 to 6,
**characterised in that** in projection along a spatial direction, the structure designed in the form of a braid, a sponge or ball structure, at least partially forms the reflective effective active surface for the ultrasonic waves propagating long the spatial direction.

8. Structure according to any one of claims 1 to 7, **characterised in that** at least one of the following substances is applied to or integrated into the structure: pharmaceutical active substance, adhesive glue, hygroscopic material.

9. Structure according to any one of claims 1 to 8,
**characterised in that** structure comprises meta materials or hybrid material combinations of meta material and biological tissue material and/or biocompatible polymer.

10. Structure according to any one of claims 3 to 9,
**characterised in that** the structure is formed of nanostructures or at least superficially applied nanostructures.

11. Structure according to claim 10,
**characterised in that** the nanostructures are in the form of nanotubes or nanograss.

12. Device for implanting the structure according to any one of claims 1 to 11, comprising the following components:
- hollow cannula adapted and suitable for the subcutaneous implantation of the structure, that in the first state is accommodated within the hollow cannula and distally can be applied intracorporeally in a subcutaneous manner from the hollow cannula,
- means for distally discharging the structure from the hollow cannula and intracorporeal application.

13. Device for implanting according to claim 2 and 12,
**characterised in that** a generator is provided for producing the magnetic, electrical, caloric and/or acoustic force field that enters into interaction with the at least one means of the structure.

14. Device for recording an intracorporeal movement pattern with the structure according to any one of claims 1 to 11, as well as a sonography device that is extracorporeally positioned in such a way that by way of ultrasonic waves generated by the sonography device which hit the effective active surface of the intracorporeally applied structure and are partially reflected thereon, spatial changes in distance between the effective active surface and the sonography device can be recorded.

15. Device according to claim 14 as well as claim 5,
**characterised in that** a transmitter and receiver device for electromagnetic fields is arranged extracorporeally, the electromagnetic field of which interacts with the RFID, interdigital electrode and/or electrical coil structure.

## Revendications

1. Structure fabriquée à partir d'au moins un matériau biocompatible, qui peut être implantée de façon transcutanée, intracorporelle par une canule creuse et qui
- est constituée à la manière d'un film, d'un tissu, d'une structure en éponge ou en pelote,
- a le pouvoir de se déployer indépendamment d'un premier état compact dans l'espace en un deuxième état s'étalant dans l'espace et déformable de façon souple,
- possède un module d'élasticité, qui correspond à celui d'une paroi vasculaire d'un vaisseau sanguin de l'ordre de grandeur se situant entre 10⁵ N m⁻² et 10⁷ N m⁻²,
- possède au moins une gamme avec une impédance acoustique supérieure à 1,63·10⁶ kg/m²s,
- possède dans le deuxième état une surface active effective, qui est déformable de façon souple et réfléchit des ondes ultrasonores au moins en partie, et
- comporte dans le premier état une dimension de diamètre, qui correspond à un diamètre intérieur d'une canule creuse avec une taille de canule se situant entre 10 G et 30 G.

2. Structure selon la revendication 1,
**caractérisée en ce que** la structure comporte au moins un moyen, qui peut interagir avec un champ de force extracorporel magnétique, électrique, calorique et/ou acoustique sonore de telle manière qu'un couple de force modifiant la forme spatiale et/ou la position spatiale de la structure peut être produit.

3. Structure selon la revendication 1 ou 2,
**caractérisé en ce que** la structure possède dans le deuxième état une surface active effective d'au moins 0,2 mm² et au maximum de 500 mm².

4. Structure selon l'une quelconque des revendications 1 à 3,
**caractérisée en ce que** la structure peut être transformée dans le premier état en partant du deuxième état par pliage, compression et/ou enroulement.

5. Structure selon l'une quelconque des revendications 2 à 4,
**caractérisée en ce que** le moyen interagissant avec le champ de force externe est composé d'au moins un matériau du groupe de matériaux suivant : matériau magnétique ou magnétisable, matériau électroconducteur, matériau bimétallique, matériau transducteur électrique ou magnétique, matériaux à mémoire de forme.

6. Structure selon l'une quelconque des revendications 1 à 5,
**caractérisée en ce que** la surface active effective est constituée sous la forme d'une surface cohérente en une pièce.

7. Structure selon l'une quelconque des revendications 1 à 6,
**caractérisée en ce que** la structure constituée à la manière d'un tissu, d'une structure en éponge ou en pelote forme en projection le long d'une direction spatiale la surface active effective réfléchissante au moins en partie pour des ondes ultrasonores se propageant le long de la direction spatiale.

8. Structure selon l'une quelconque des revendications 1 à 7,
**caractérisée en ce qu'**au moins une des substances suivantes est appliquée à la structure ou intégrée dans celle-ci : agent actif pharmaceutique, adhésif d'adhérence, matériau hygroscopique.

9. Structure selon l'une quelconque des revendications 1 à 8,
**caractérisée en ce que** la structure comporte des métamatériaux ou des combinaisons de matériaux hybrides de métamatériau et de matériau tissulaire biologique et/ou de polymère biocompatible.

10. Structure selon l'une quelconque des revendications 3 à 9,
**caractérisée en ce que** la structure est constituée de nanostructures ou au moins avec des nanostructures retenues en surface.

11. Structure selon la revendication 10,
**caractérisée en ce que** les nanostructures sont constituées sous la forme de nanotubes ou de nanoherbe.

12. Dispositif pour implanter la structure selon l'une quelconque des revendications 1 à 11, comprenant les composants suivants :
- canule creuse adaptée et convenant à l'implantation sous-cutanée de la structure, qui est logée en premier état à l'intérieur de la canule creuse et peut être appliquée de façon intracorporelle sous-cutanée du côté distal depuis la canule creuse,
- moyen pour la diffusion du côté distal de la structure depuis la canule creuse et pour l'application intracorporelle.

13. Dispositif pour l'implantation selon les revendications 2 et 12,
**caractérisé en ce qu'**un générateur est prévu pour la production du champ de force magnétique, électrique, calorique et/ou acoustique sonore, qui interagit avec au moins un moyen de la structure.

14. Dispositif pour détecter un modèle de mouvement intracorporel à l'aide de la structure selon l'une quelconque des revendications 1 à 11 et un appareil d'échographie, qui est positionné de façon extracorporelle de telle sorte qu'au moyen des ondes ultrasonores produites par l'appareil d'échographie, qui rencontrent la surface active effective de la structure appliquée de façon intracorporelle et réfléchissent en partie sur celle-ci, il est possible de détecter des variations de distance spatiales entre la surface active effective et l'appareil d'échographie.

15. Dispositif selon la revendication 14 et la revendication 5,
**caractérisé en ce qu'**une unité émettrice et réceptrice pour des champs électromagnétiques est disposée de façon extracorporelle, dont le champ électromagnétique interagit avec la structure d'identification par radiofréquence (RFID), à électrodes intra-numériques et/ou à bobine électrique.
